(19) Europäisches Patentamt
European Patent Office
Office européen des brevets

(11) Publication number: 0 079 167 A1

# (12) EUROPEAN PATENT APPLICATION

(21) Application number: 82305687.4

(22) Date of filing: 27.10.82

(51) Int. Cl.$^3$: C 07 D 487/04
A 61 K 31/40
//(C07D487/04, 209/00, 205/00)

(30) Priority: 09.11.81 GB 8133769

(43) Date of publication of application: 18.05.83 Bulletin 83/20

(84) Designated Contracting States: BE CH DE FR GB IT LI NL SE

(71) Applicant: BEECHAM GROUP PLC
Beecham House Great West Road
Brentford Middlesex(GB)

(72) Inventor: Coulton, Steven
6 Badgers Close
Horsham West Sussex(GB)

(72) Inventor: Corbett, David Francis
72E Holmesdale Road
Reigate Surrey(GB)

(74) Representative: Hesketh, Alan, Dr.
European Patent Attorney Beecham Pharmaceuticals
Great Burgh Yew Tree Bottom Road
Epsom, Surrey, KT18 5XQ(GB)

## (54) A carbapenem compound, preparation and use.

(57) The compound of formula (II):

(II) 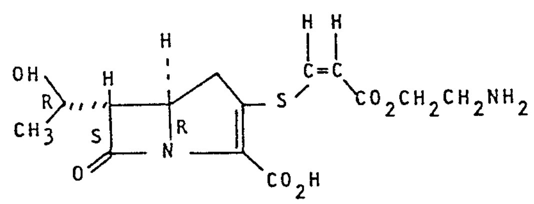

namely (5R, 6S)-3-[2-(Z)-(2-aminoethoxycarbonyl)ethenylthio]-6-[(R)-1-hydroxyethyl]-7-oxo-1-azabicyclo[3.2.0]-hept-2-ene-2-carboxylic acid; is useful in the treatment of a bacterial infection in animals and is preferably administered as a pharmaceutical formulation in association with a pharmaceutically acceptable carrier. The compound may be prepared by, e.g., reaction of the thiol with a compound of formula (V):

$$H-C\equiv C-CO_2CH_2CH_2X \qquad (V)$$

wherein X is amino or a precursor thereof.

EP 0 079 167 A1

0079167



TITLE MODIFIED
see front page

NOVEL COMPOUNDS, THEIR PREPARATION AND USE

This invention relates to novel 7 - oxo - 1 - azabicyclo [3.2.0] heptene derivatives and in particular to such derivatives with C-3 carboxyvinylthio substituents. This invention further relates to processes for their preparation and to compositions containing them. These derivatives are of use in the treatment of bacterial infection.

European Patent Application Publication No: 0024832 discloses *inter alia* compounds of formula [I] :

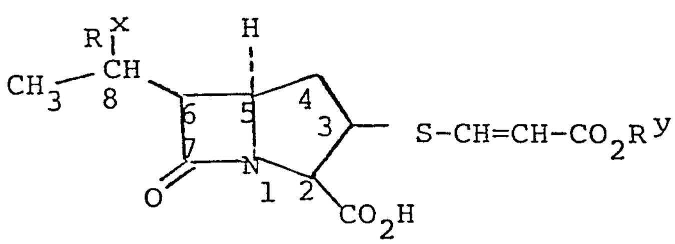

(I)

and pharmaceutically acceptable salts and cleavable esters thereof wherein $R^x$ is -OH or $-OSO_3H$ or a pharmaceutically acceptable salt or $C_{1-4}$ alkyl ester thereof, and $R^y$ represents a hydrogen atom, a pharmaceutically acceptable salting-ion or a $C_{1-4}$ alkyl or amino $C_{1-4}$ alkyl group.

We have now found that one compound within formula (I), (5R,6S)-3-[2-(Z)-(2-aminoethoxycarbonyl)-ethenylthio]-6-[(R)-1-hydroxyethyl]-7-oxo-1-azabicyclo

[3.2.0] hept-2-ene-2-carboxylic acid, shows superior efficacy, for example antibacterial activity and/or stability, to closely related compounds specifically disclosed in the aforementioned European Patent Application. In particular, the compound of the present invention shows an unexpected and significant improvement in antibacterial activity over the products of Examples 18 and 20 of the aforementioned European Patent Application, namely the (5R,6S) and (5R,6R) 3-[2-(Z)-(2-aminoethoxycarbonyl) ethenylthio]-6-[(S)-1-hydroxyethyl]-7-oxo-1-azabicyclo[3.2.0] hept-2-ene-2-carboxylic acids.

Accordingly, the present invention provides the compound of formula (II):

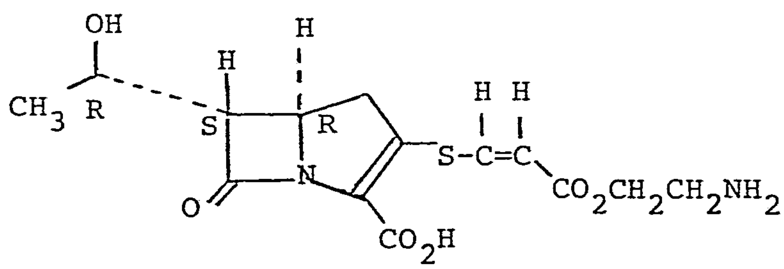

(II)

having 8R,6S,5R stereochemistry, and <u>cis</u>-stereochemistry in the double bond of the C-3 side chain.

The compound of formula (II) is most suitably presented in the form of a zwitterion; that is:

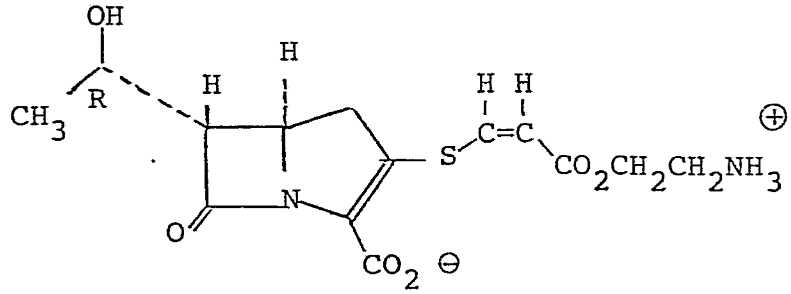

(III)

In another aspect the present invention provides a process for the preparation of a compound of formula (II), which process comprises the reaction, in the presence of an acid acceptor, of a cleavable ester. of a compound of formula (IV):

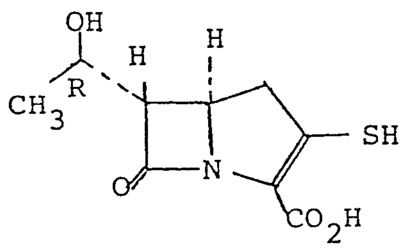

(IV)

with a compound of formula (V) :

$$H-C \equiv C-CO_2\ CH_2\ CH_2\ X \qquad (V)$$

wherein X is an amino group or a precursor thereof; and any amino or hydroxy group is optionally protected; and thereafter cleaving the cleavable ester and removing any protecting groups.

Suitable precursors of the amino group include the azido and nitro groups which are convertible to amino in a conventional manner.

In one embodiment, the C-8 Hydroxy group of the compound of formula (IV) may be protected as a formate.

Suitable acid acceptors are carbonates and bicarbonates such as anhydrous potassium carbonate. The reaction is generally performed in a dry polar solvent such as dimethylformamide. Suitably, the reaction is performed at a non-extreme temperature, for example, -30°C to +60°C; more suitably -10°C to +40°C; and preferably at ambient temperature.

The compounds of formula (IV) above and (VI) and (VII) below may be prepared by the methods of European Patent Application No. 0024832.

In another aspect, the present invention provides a process for the preparation of a compound of the formula (II), which process comprises the hydrolysis of a compound of formula (VI) :

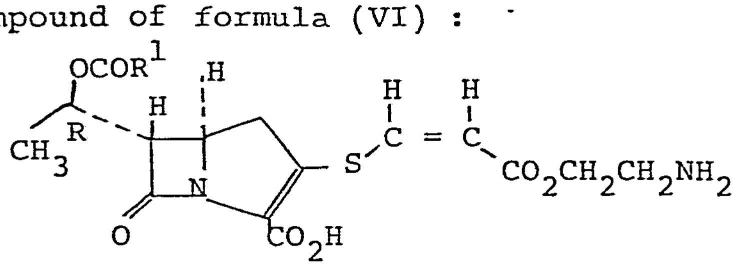

(VI)

or salt or cleavable ester thereof, wherein $R^1$ is a hydrogen atom, or a $C_{1-6}$ alkyl, aryl or aryl $C_{1-6}$ alkyl group;

and thereafter cleaving any cleavable ester.

The hydrolysis may be performed by a standard method, for example, using an alkali or alkaline earth metal salt in aqueous solution. Suitable salts include sodium hydroxide, sodium bicarbonate, potassium bicarbonate, sodium carbonate, potassium carbonate and potassium hydroxide. Of these, sodium hydroxide is preferred when the C-2 carboxy group is esterified.

Suitable salts for the compounds of the formula (VI) include inorganic salts, for example metal salts such as silver or mercuric salts, or alkali metal salts such as lithium or sodium salts, and organic salts such as tertiary amine salts.

When $R^1$ is or includes aryl, a preferred aryl group is phenyl.

The compounds of formula (VI) may be prepared by the reaction of a cleavable ester of a compound of formula (VII):

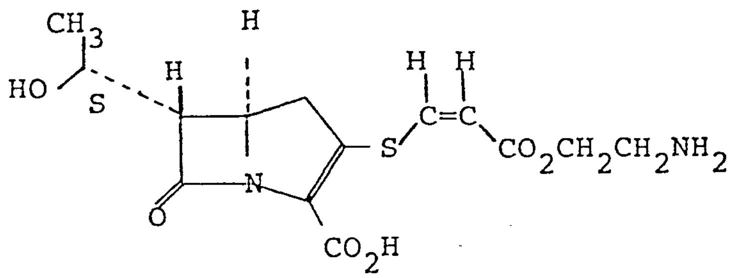

(VII)

having 8S stereochemistry, (i.e. inverted compared with that at C-8 of the compound of formula (II)); with

(a) a compound of formula (VIII) :

$R^1CO_2H$ (VIII)

wherein $R^1$ is as hereinbefore defined;

(b) a compound of formula (IX) :

$R^2O.\ CO.N = N.\ CO.\ OR^3$ (IX)

wherein $R^2$ and $R^3$ are independently $C_{1-6}$ alkyl, aryl-$C_{1-6}$ alkyl or aryl; and

(c) a compound of formula (X):

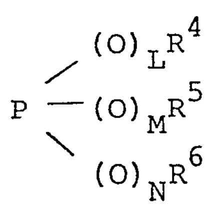

(X)

wherein L, M and N are independently zero or one and $R^4$, $R^5$ and $R^6$ are independently $C_{1-6}$ alkyl, aryl $C_{1-6}$ alkyl or aryl;

and thereafter, if necessary, cleaving a cleavable ester.

A preferred compound of formula (VIII) is formic acid.

Suitable compounds of formula (IX) include those wherein $R^2$ and $R^3$ are independently selected from methyl, ethyl, propyl, butyl, benzyl and phenyl. It is generally convenient that $R^2$ and $R^3$ each represent the same moiety.

Particularly suitable compounds of formula (IX) include those wherein $R^2$ and $R^3$ each represent an ethyl, t-butyl or isopropyl group.

Suitable compounds of formula (X) include those wherein the $R^4$, $R^5$ and $R^6$ groups are selected from methyl, ethyl, n-propyl, n-butyl, benzyl, phenyl and methoxyphenyl groups. It is generally convenient that $R^4$,$R^5$ and $R^6$ each represent the same moiety.

Favoured compounds of formula (X) include tri-aryl phosphines and tri-alkyl phosphites. Particularly suitable compounds of the formula (X) include triphenylphosphine, trimethylphosphite and triethylphosphite, of these triphenylphosphine is preferred.

In general, in the reaction of the compounds (VII) - (X), it has proved convenient to use one equivalent of the compound of formula (VII), approximately two equivalents of the compounds of formulae (IX) and (X), and from two to five equivalents of the compound of formula (VIII). The reaction is performed in an inert organic solvent which should be aprotic and substantially unreactive towards the reagents involved. Suitable solvents include tetrahydrofuran, dioxan,

1,2-dimethoxyethane, benzene, toluene and mixtures thereof. The reaction is normally performed at a non-extreme temperature such as $-60^{o}C$ to $+100^{o}C$, more suitably from about $-10^{o}C$ to $+50^{o}C$, and most conveniently at ambient temperature.

In another aspect the present invention provides a process for the preparation of a compound of formula (II), which process comprises the reaction of a compound of formula (XI) :

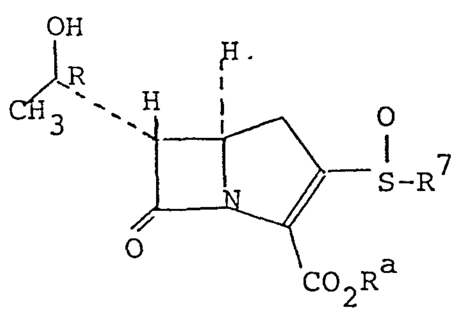

(XI)

wherein $R^a$ is a carboxy-protecting group; and $R^7$ is $C_{1-6}$ alkyl, $C_{3-7}$ cycloalkyl, $C_{3-7}$ cycloalkyl $C_{1-6}$ alkyl, aryl$C_{1-6}$alkyl, heterocyclyl $C_{1-6}$ alkyl, heteroaryl $C_{1-6}$ alkyl, heterocyclyl, heteroaryl, aryl, $C_{2-6}$ alkenyl or $C_{2-6}$ alkynyl group, any of groups $R^7$ being optionally substituted;

with a compound of formula (XII) :

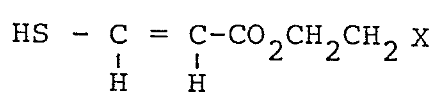

(XII)

or reactive derivative thereof, wherein X is an amino group or a precursor thereof; and any amino or hydroxy group is optionally protected;

and thereafter removing any protecting group(s), such as the carboxy-protecting group.

Suitably the C-8 hydroxy group is protected as a formate.

This reaction may be performed in any solvent that is substantially inert during the reaction, for example, tetrahydrofuran, dimethylformamide, dioxan, hexamethyl phosphoramide, dimethoxyethane or dimethoxydiethyl ether. Of these solvents dimethylformamide is preferred.

Alternatively, we have found it useful to use a phase transfer catalyst. Particularly suitable phase transfer catalysts include tetra-n-butyl ammonium bromide, cetylbromide and cetyltriethylammonium chloride; suitable solvents include halogenated water-immiscible solvents such as chloroform in the presence of water.

The reaction is normally performed at ambient or a depressed temperature, for example $20^{o}C$ to $-70^{o}C$, and preferably between $0^{o}C$ and $-50^{o}C$. However, when using a phase transfer catalyst it is preferable to conduct the reaction between $0^{o}C$ and ambient temperature.

When the thiol of formula (XII) is used, the reaction is normally carried out in the presence of a base. Examples of such bases include sodium hydride, sodium hydroxide, sodium alkoxide such as methoxide, ethoxide or butoxide, sodium amide, potassium hydroxide, potassium alkoxide such as methoxide ethoxide or butoxide, potassium amide, and trialkylamines such as triethylamine and tri-n-propylamine. Of these, triethylamine is preferred. Preferably, the base is present in an amount of at least 0.9 equivalents, more preferably between 1.0 and 1.2 equivalents per mole of the thiol compound. Instead of using a base in the reaction, a reactive derivative of the thiol may be used, preferably the reactive derivative is a salt of the thiol, in particular an alkali metal salt such as sodium or potassium. The amount of thiol compound of the formula (XII) or reactive

derivative thereof is generally between 1.0 and 1.5 moles per mole equivalent of the compound of the formula (XI).

Suitable carboxy-protecting derivatives for the group $CO_2R^a$ include salts, esters and anhydride derivatives of the carboxylic acid. The derivative is one which may readily be cleaved at a later stage of the reaction. Salts need not be pharmaceutically acceptable.

When $R^7$ is or includes aryl, a preferred aryl group is phenyl; a preferred heterocyclyl group is tetrahydrofuranyl; and a preferred heteroaryl group is thienyl.

In another aspect the present invention provides a process for the preparation of a compound of formula (II), which process comprises the elimination of the elements of $R^8R^9R^{10}P=O$ from a cleavable ester of a compound of formula (XIII) :

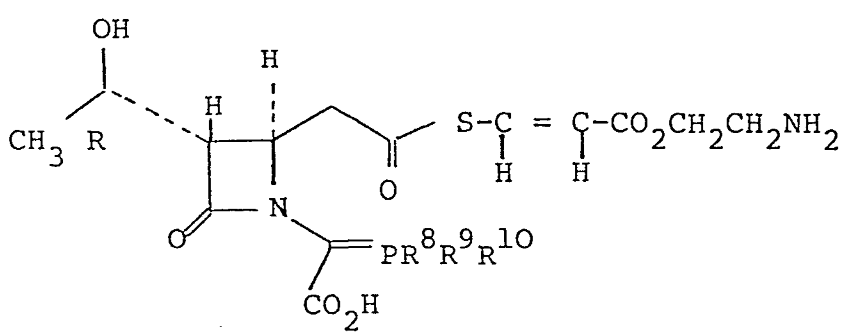

(XIII)

wherein $R^8$, $R^9$ and $R^{10}$ are independently phenyl or methoxyphenyl, and wherein any amino or hydroxy group is optionally protected;
and thereafter cleaving the cleavable ester.
Suitably, this process is performed by heating the ester of the compound of formula (XIII) in an inert organic solvent, for example, in a solvent such as toluene, preferably under substantially anhydrous conditions. Temperatures of 90°C to 120°C and more suitably 100°C to 110°C may be employed.

The compounds of formula (XIII) may be prepared by methods analogous to those disclosed in European Patent Applicaton Publication Nos: 0008514 and 0003740.

In the processes of this invention any amino group present can be conveniently protected in conventional manner, for example as a p-nitrobenzyloxycarbonyloxyamino group.

Suitable cleavable esters of the compounds of formulae (IV), (VI), (VII) and (VIII) include those convertible by chemical or biologicals methods to the free acid, such as by <u>in-vivo</u> hydrolysis, enzymic hydrolysis, hydrogenolysis, hydrolysis, electrolysis and photolysis.

Suitably, the carboxylic acid is esterified by a group of sub-formulae (a) - (f) :

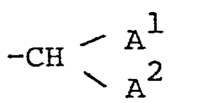
(a)

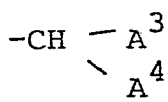
(b)

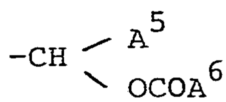
(c)

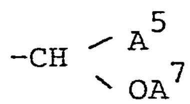
(d)

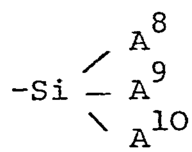
(e)

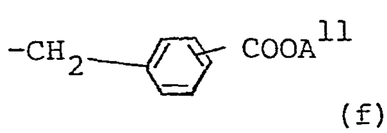
(f)

wherein $A^1$ is a hydrogen atom, a $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group, or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenyl or bromophenacyl group.

Favourably, $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group, or is joined to $A^5$. Favourably, $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable in-vivo hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formula (II) or its salt.

Suitable esters of this type include those of sub-formula (c) as hereinbefore defined.

When used in the treatment of a bacterial infection, the compound of formula (II) is preferably administered in the form of a pharmaceutical formulation.

Therefore in a further aspect, the present invention also provides a pharmaceutical composition which comprises a compound of formula (II) and a pharmaceutically acceptable carrier therefor.

The compositions of this invention may be prepared by conventional methods of preparing antibiotic compositions and in conventional manner may be adapted for oral, topical or parenteral administration.

The compositions of this invention are conveniently in the form of a unit-dose composition adapted for oral administration.

Alternatively, the compositions of this invention are in the form of a unit dose composition adapted for administration by injection.

Unit-dose forms according to this invention will normally contain from 50 to 500 mgs of a compound of this invention, for example about 62.5, 100, 125, 150, 200, 250, or 300 mgs. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The carriers used in the compositions of this invention may include diluents, binders, disintegrants, lubricants, colours, flavouring agents and preservatives in conventional manner. Thus suitable agents include lactose, starch, sucrose, calcium phosphate, sorbitol,polyvinylpyrrolidone, acacia, gelatin, tragacanth or the like, potato starch or polyvinylpolypyrrolidone, magnesium stearate and sodium lauryl sulphate.

Orally administrable forms of the compositions of this invention are most suitably in the form of unit-dose units such as tablets or capsules.

The compounds and compositions of this invention may be used in the treatment of a bacterial infection in animals, such as mammals including humans, for example, infections of the respiratory tract, urinary tract or soft tissues, or mastitis in cattle. Such compositions may be administered to susceptible gram-positive or gram-negative bacteria such as strains of *Staphylococcus aureus*, *Klebsiella aerogenes* and *Escherichia coli*.

Therefore, in a further aspect, the present invention provides a method for the treatment of a bacterial infection in an animal, which method comprises the administration to said animal of a non-toxic, antibacterially effective amount of a compound of formula (II) or a pharmaceutical formulation thereof.

The present invention will now be illustrated by the following Examples and data.

Example 1

a) p-Nitrobenzyl (5R,6S)-3-[(Z)-2-(2-N-p-nitrobenzyloxycarbonylamino-ethyloxycarbonyl)ethenylthio]-6-[(R)-1-hydroxyethyl]-7-oxo-1-azabicyclo[3.2.0]hept-2-ene-2-carboxylate.

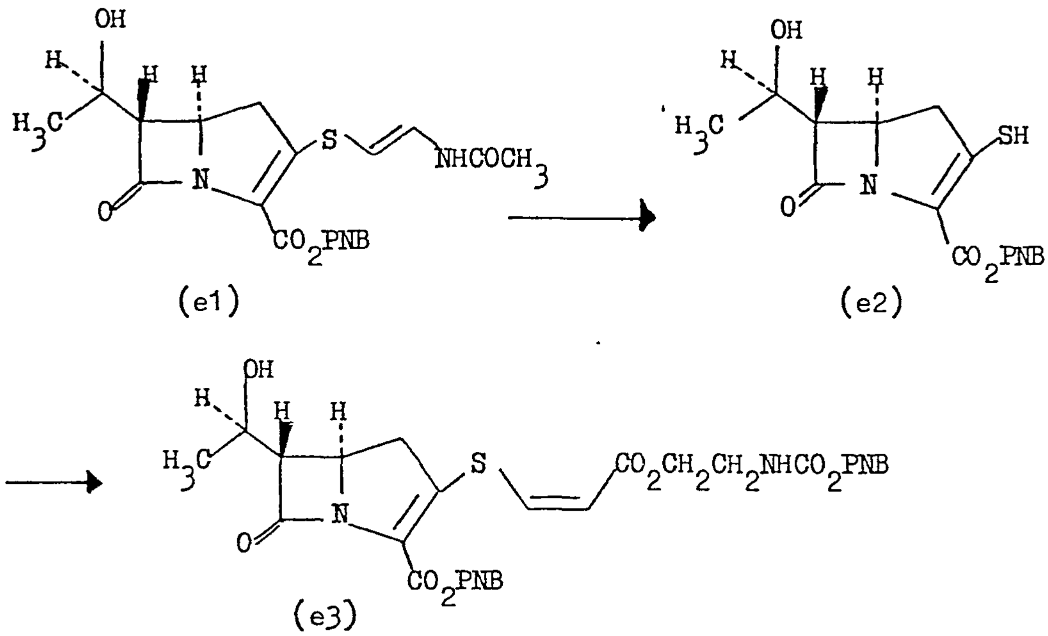

(e1) (e2) (e3)

p-Nitrobenzyl (5R,6S)-3-[(E)-2-acetamidoethenylthio]-6-[(R)-1-hydroxyethyl]-7-oxo-1-azabicyclo[3.2.0]hept-2-ene-2-carboxylate (e1) (100 mg) was dissolved in 1,4-dioxan (5 ml) containing water (10 drops) and stirred at room temperature for 5 minutes with N-bromoacetamide (31 mg). Chloroform was added and the organic phase was washed with pH 7.0, 0.05 M phosphate buffer, saturated sodium chloride solution and dried over anhydrous magnesium sluphate. Filtration and removal of the solvent at reduced pressure afforded the crude thiol (e2), as a pale yellow oil.

The crude thiol (e2) was dissolved in dry dimethylformamide (5 ml) and stirred at room temperature for 15 minutes with 2-(p-nitro benzyloxycarbonylamino)ethyl propionate (80 mg) and anhydrous potassium carbonate (31 mg). Ethyl acetate was added and the organic solution washed with water, saturated sodium chloride solution and dried over anhydrous magnesium sulphate. After filtration, the solvent was removed at reduced pressure to yield an oil, which was

chromatographed over silica gel (14 gm). Elution with a gradient of 0 to 2% ethanol/chloroform gave the title compound as a white solid (40 mg), $\nu_{max.}$ (KBr) 3410, 1775, 1720 (shoulder), 1700, 1603, 1520 $cm^{-1}$., $\lambda_{max.}$ (EtOH) 335 nm (Em 10650), 265 nm (Em 11710), $\delta_H$ ($d_7$ -DMF) 1.26 (3H, d, C$\underline{H}_3$CH), 3.4 - 3.8 (m, 4-$CH_2$ + 6-C$\underline{H}$ + C$\underline{H}_2$), 4.12 (1H, broad quintet, 8-C$\underline{H}$), 4.28 (2H, m, C$\underline{H}_2$), 4.40 (1H, dt, 5-C$\underline{H}$), 5.27 (2H, s, C$\underline{H}_2$ Ar), 5.50 (2H, AB, C$\underline{H}_2$Ar), 6.10 (1H, d, J 9.3 Hz, cis C$\underline{H}$=C), 7.60 (1H, broad t, exchanges with $D_2O$, N$\underline{H}$), 7.67 (2H, d, aromatic protons), 7.75 - 8.00 (3H, m, C$\underline{H}$=C & aromatic protons), 8.23 - 8.36 (4H, 2 x d, aromatic protons).

b) <u>(5R,6S)-3-[(Z)-2-(2-aminoethyloxycarbonyl)ethenylthio]-6-[(R)-1-hydroxyethyl]-7-oxo-1-azabicyclo[3.2.0]hept-2-ene-2-carboxylic acid</u>

(e3) 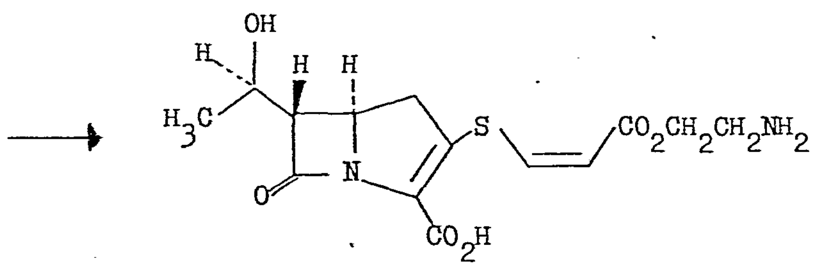

(e4)

The ester (e3) (35 mg) was dissolved in 1,4-dioxan (20 ml), pH 7,0 0.05 M phosphate buffer (3.75 ml) and water (3 ml) and shaken with hydrogen at ambient temperature and pressure for 2 hours in the presence of 5% palladium on charcoal catalyst (60 mg). The reaction solution was filtered over Celite, washing well with water. The filtrate was then evaporated to smaller volume at reduced pressure and washed with ethyl acetate (3 x 50 ml). The solution was then chromatographed over Hp-20, eluting with a gradient of 0 - 20% ethanol/water. The column fractions were monitored by u.v. and those possessing the desired absorption ($\lambda_{max}$ 324 nm) were combined The resulting solution was estimated to contain 4.7 mg title compound (e4) based on Em 17,000 at $\lambda_{max}$ 324 nm in the u.v. spectrum.

Example 2

a) p-Nitrobenzyl (5R,6S)-3-[2-(2-p-nitrobenzyloxycarbonylamino-ethoxycarbonyl)ethenylthio]-6-[(R)-1-formyloxyethyl]-7-oxo-1-azabicyclo[3.2.0]hept-2-ene-2-carboxylate

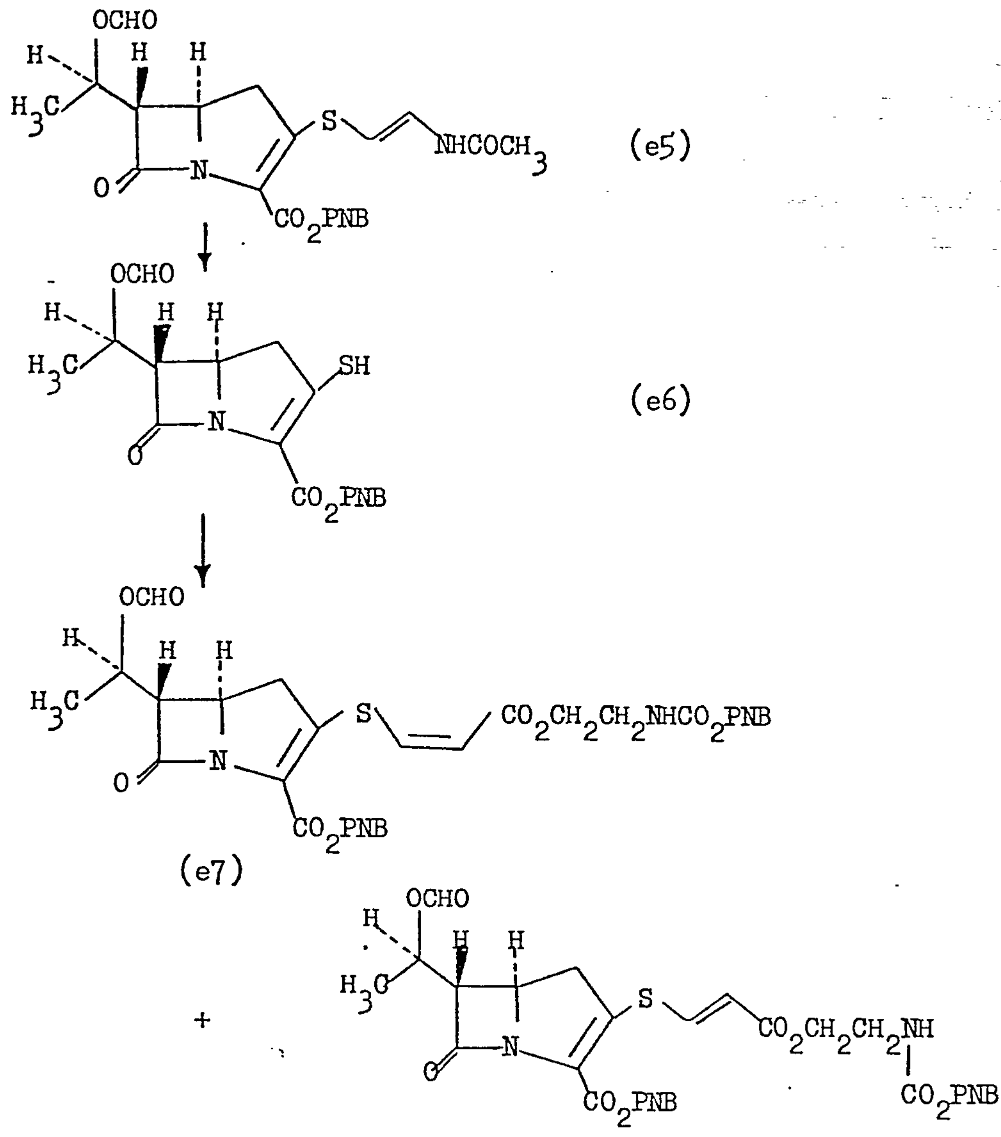

(e5)

(e6)

(e7)

+

(e8)

p-Nitrobenzyl (5R,6S)-3-(E-2-acetamidoethenylthio)-6-[(R)-1-formyloxyethyl]-7-oxo-1-azabicyclo[3.2.0.]hept-2-ene-2-carboxylate (e5) (377 mg) was dissolved in aqueous 1,4-dioxan (50 ml) and stirred at room temperature for 5 minutes with N-bromoacetamide (109.5 mg). Chloroform was added and the organic phase was washed with pH 7.0,

0.05 M phosphate buffer, saturated sodium chloride solution and dried over anhydrous magnesium sulphate. Filtration and removal of the solvent at reduced pressure afforded the crude thiol (e6) as a pale yellow oil, $\nu_{max.}$ ($CHCl_3$) 1781, 1723, 1610, 1560, 1525, 1355, 1340 $cm^{-1}$.

The crude thiol (e6) was dissolved in dry dimethylformamide (10 ml) and stirred at room temperature for 15 minutes with 2-(p-nitrobenzyloxycarbonylamino)ethyl propionate (350 mg) and anhydrous potassium carbonate (109.5 mg). Ethyl acetate was added and the organic solution washed with water, saturated sodium chloride solution and dried over anhydrous magnesium sulphate. After filtration, the solvent was removed at reduced pressure to yield a yellow oil, which was chromatographed over silica gel (20 gm). Elution with ethyl acetate afforded the crude product as a pale yellow foam (310 mg). This foam was re-chromatographed over silica gel (15 gm), eluting with chloroform. The resulting pale yellow oil, on trituration with diethyl ether afforded a pale yellow solid (155 mg). This consisted of p-nitrobenzyl (5R,6S)-3-[2-(2-p-nitrobenzyloxycarbonylaminoethoxycarbonyl)ethenylthio]-6-[(R)-1-formyloxyethyl]-7-oxo-1-azabicyclo [3.2.0]hept-2-ene-2-carboxylate as a mixture of the Z-isomer (e7) and the E-isomer (e8) in the approximate ratio 9:1; $\lambda_{max.}$ (EtOH) 334 nm (Em 17,746), 265 nm (Em 18, 579), $\nu_{max.}$ ($CHBr_3$) 3400, 1781, 1720, 1605, 1520, 1345 $cm^{-1}$, $\delta_H$ ($CDCl_3$) 1.47 (3H, d, $C\underline{H}_3CH$), 3.24 (1H, dd, 4-C$\underline{H}$a), 3.4-3.6 (4H, m, $C\underline{H}_2NH$ + 6-C$\underline{H}$ + 4-C$\underline{H}$b), 4.30 (3H, m, $CO_2C\underline{H}_2$ + 5-C$\underline{H}$), 5.20 (2H, s, $C\underline{H}_2Ar$), 5.22-5.58 (4H, q + m, $C\underline{H}_2AR$ + 8-C$\underline{H}$ + N$\underline{H}$), 6.00 (1H, d, J 10.5 Hz, cis C$\underline{H}$=C), 7.26 (1H, d, J 10.5Hz, cis C$\underline{H}$=C), 7.49 (2H, d, aromatic protons), 7.66 (2H, d, aromatic protons), 8.07 (1H, s, C$\underline{H}$O), 8.13 - 8.30 (4H, 2 x d, aromatic protons). The E-isomer shows inter alia δ 6.12 (d, J 15 Hz, trans C$\underline{H}$=C), 7.77 (d, J 15 Hz, trans C$\underline{H}$=C).

b) <u>(5R,6S)-3-[(Z)-2-(2-aminoethyloxycarbonyl)ethenylthio]-6-[(R)-1-formyloxyethyl]-7-oxo-1-azabicyclo[3.2.0]hept-2-ene-2-carboxylic acid (e9) and (5R,6S)-3-[(Z)-2-(2-aminoethyloxycarbonyl)ethenylthio]-6-[(R)-1-hydroxyethyl]-7-oxo-1-azabicyclo[3.2.0]hept-2-ene-2-carboxylic acid (e4)</u>

(e7) + (e8)

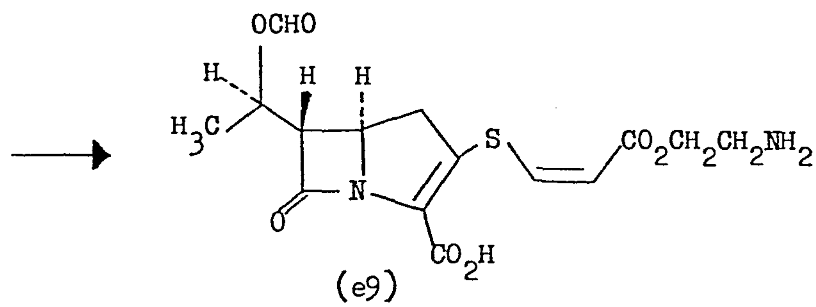

(e9)

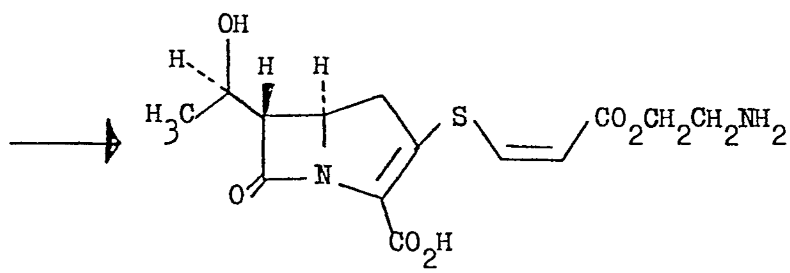

(e4)

The mixture of esters (e7) and (e8) (500 mg) was dissolved in 1,4-dioxan (75 ml), water (23 ml) and pH 7.0, 0.05 M phosphate buffer (23 ml) and shaken with hydrogen in the presence of 5% palladuim on carbon catalyst (750 mg) at ambient temperature and pressure for 3.5 hours. The solution was filtered over Celite, washing well with water, and evaporated to smaller volume at reduced pressure. The filtrate was washed with ethyl acetate (3 x 250 ml) and evaporated again at reduced pressure in order to remove ethyl acetate. The resulting solution, which was estimated to contain 125 mg of the formyloxy derivative (e9) based on Em 17,000 at $\lambda_{max.}$ 325 nm in the u.v. spectrum, was stirred at pH 9.0 and room temperature for 3.5 hours. The pH was then readjusted to 7.0 and the solution evaporated to small volume at reduced pressure. Chromatography over HP-20, eluting with a gradient of 0 to 5% ethanol/water afforded an aqueous solution containing the pure (5R, 6S)-3-[(Z)-2-(2-amino-

ethyloxycarbonyl)ethenylthio]-6-[(R)-1-hydroxyethyl]-7-oxo-1-azabicyclo[3.2.0]hept-2-ene-2-carboxylic acid (e4). Evaporation to small volume followed by lyophilisation yielded the product as a white fluffy solid (40 mg), $\lambda_{max}$ ($H_2O$) 325 nm (Em 10,550).

In another experiment, the crude formyloxy derivative (e9), obtained by hydrogenolysis of the mixture of esters (e7) and (e8), was purified by chromatography over HP-20, eluting with a gradient of 0 to 20% ethanol/water. Lyophilisation afforded (5R,6S)-3-[(Z)-2-(2-aminoethyloxycarbonyl)ethenylthio]-6-[(R)-1-formyloxyethyl]-7-oxo-1-azabicyclo[3.2.0]hept-2-ene-2-carboxylic acid (e9) as a white fluffy solid, $\lambda_{max}$ ($H_2O$) 325 nm (Em 15,033). This sample contained a small amount of the E-isomer (<5% by h.p.l.c.), which could be removed, if desired, by further chromatography.

Demonstration of Effectiveness

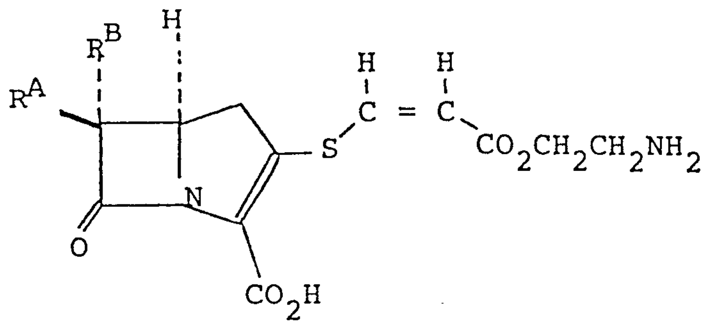

stereochemistry

Compound A: $R^A$= $CH_3$ 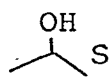 S $R^B$ = H 8S,6R,5R

Compound B: $R^A$= H $R^B$ = $CH_3$ 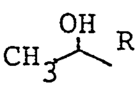 R 8R,6S,5R

Compound C: $R^A$= H $R^B$ = $CH_3$ 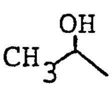 S 8S,6S,5R

Antibacterial Activity in vitro (MIC µg/ml)

Compound A

Organism

| | | |
|---|---|---|
| Escherichia coli | JT4R+ | 6.2 |
| " " | JT2OR+ | 6.2 |
| " " | E96R+ | 0.8 |
| " " | Ba78R+ | 1.6 |
| Klebsiella aerogenes | VA2R+ | 25 |
| " " | Ba95R+ | 6.2 |
| " " | 185R+ | 3.1 |
| " " | 192R+ | 6.2 |
| Staphylococcus aureus | ATCC 25923 | 0.1 |

| | | |
|---|---|---|
| Staphylococcus aureus | T100 | 0.2 |
| " " | Smith | 0.2 |
| " " | 150+ | 0.2 |
| " " | MB9+ | 0.2 |
| " " | T71+ | 0.1 |
| " " | N71MR | 0.8 |
| " " | N36MR | 0.4 |
| " " | 68/8484 | 0.2 |

Compound B

| | | |
|---|---|---|
| Escherichia coli | JT4R+ | 0.1 |
| " " | JT56R+ | 0.2 |
| " " | JT177R | 0.2 |
| " " | Ba78R+ | 0.1 |
| Klebsiella aerogenes | Va2R+ | 0.4 |
| " " | Ba95R+ | 0.4 |
| " " | Du185R | 0.2 |
| " " | Du192R | 0.2 |
| Staphylococcus aureus | T2p- | 0.1 |
| " " | T100p- | 0.02 |
| " " | T120p- | 0.02 |
| " " | T97p+ | 0.1 |
| " " | T118p+ | 0.05 |
| " " | MANSTK 33269 | 0.4 |
| " " | N36R | 0.1 |

Compound C

| | | |
|---|---|---|
| Escherichia coli | JT39 | 3.1 |
| Klebsiella aerogenes | A | 3.1 |
| Staphylococcus aureus | Russell | 0.4 |
| Staphylococcus aureus | 1517 | 6.2 |

Tissue Stability Values

A preparation of 2% mouse kidney homogenate was mixed with equal volumes of a 200 µg/ml solution of the antibiotic to be tested. The stability of the antibiotic at 37°C was studied by removal of aliquots and subjecting them to a high pressure liquid chromatographic analysis. The values are given in figures relative to that obtained for MM13902 of U.K. Patent No. 1489235. The values are given in figures relative to that obtained for MM13902 which is taken as standard (1.0). Values greater than 1.0 reflect the fold increase in stability relative to MM13902.

| Tissue | Compound A | Compound B | MM13902 |
|---|---|---|---|
| Mouse Kidney | 1.3 | 2.4 | 1.0 |
| Human Kidney | 1.2 | 2.2 | 1.0 |

Antibacterial Activity in-vivo against Experimental Mouse infections

$CD_{50}$ mg/kg (total dose) - Compounds dosed at 1 and 5 hours past infection.

| Organism | Compound A | Compound B |
|---|---|---|
| Staph. aureus Smith | 4.5 | <0.4 |
| E. coli E96R+ | 66 | 13.0 |

1. The compound of formula (II):

(II)

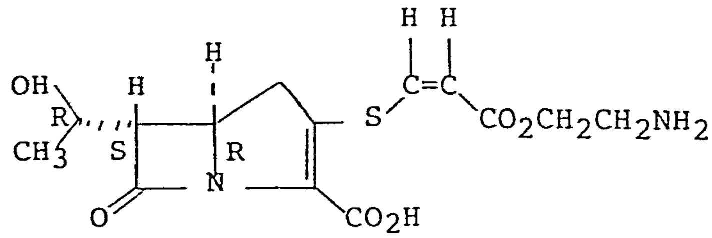

namely (5R,6S)-3-[2-(Z)-(2-aminoethoxycarbonyl)ethenylthio]-6-[(R)-1-hydroxyethyl]-7-oxo-1-azabicyclo[3.2.0]-hept-2-ene-2-carboxylic acid.

2. A pharmaceutical composition comprising the compound of formula (II) as defined in claim 1 and a pharmaceutically acceptable carrier therefor.

3. A composition according to claim 2, characterised by being adapted for oral administration.

4. A composition according to claim 3, characterised by being in the form of a tablet or a capsule.

5. A composition according to any of claims 2 to 5, characterised in that it contains from 50 to 500 mgs of the compound of formula (II).

6. A process for the preparation of the compound of formula (II) as defined in claim 1, which process comprises:

(a) the reaction, in the presence of an acid acceptor, of a cleavable ester of a compound of formula (IV):

0079167

(IV) 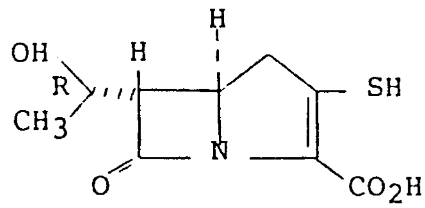

with a compound of formula (V):

$$H-C\equiv C-CO_2CH_2X \qquad (V)$$

wherein X is an amino group or a precursor thereof; and any amino or hydroxy group is optionally protected; or

(b) the hydrolysis of a compound of formula (VI) or salt or cleavable ester thereof:

(VI) 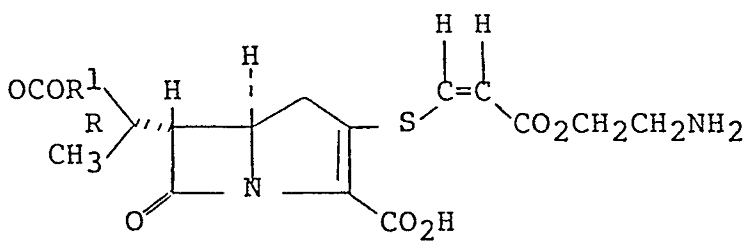

wherein $R^1$ is a hydrogen atom, or a $C_{1-6}$ alkyl, aryl or aryl $C_{1-6}$ alkyl group; or

(c) the reaction of a compound of formula (XI)

(XI) 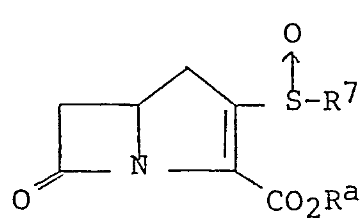

wherein $R^a$ is a carboxy-protecting group; and $R^7$ is $C_{1-6}$ alkyl, $C_{3-7}$ cycloalkyl, $C_{3-7}$ cycloalkyl $C_{1-6}$ alkyl, aryl $C_{1-6}$ alkyl, heterocyclyl $C_{1-6}$ alkyl, heteroaryl $C_{1-6}$ alkyl, heterocyclyl, heteroaryl, aryl, $C_{2-6}$ alkenyl or $C_{2-6}$ alkynyl group, any of groups $R^7$ being optionally substituted;

with a compound of formula (XII):

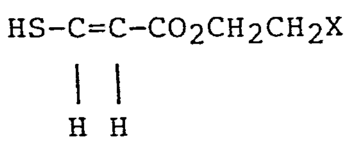

(XII)

or reactive derivative thereof, wherein X is an amino group or a precursor thereof, and any amino or hydroxy group is optionally protected; or

(d) the elimination of the elements of $R^8R^9R^{10}P=O$ from a cleavable ester of a compound of formula (XIII):

(XIII)

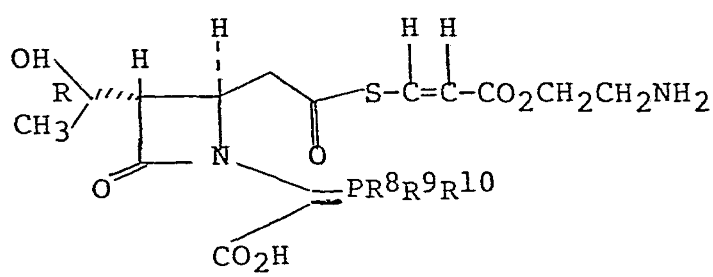

wherein $R^8$, $R^9$ and $R^{10}$ are independently phenyl or methoxyphenyl, and wherein any amino or hydroxy group is optionally protected;

and thereafter, where necessary, cleaving the cleavable ester and removing any protecting group.

7. A process according to claim 6(a), characterised in that the cleavable ester in the p-nitrobenzyl ester and X is p-nitrobenzyloxycarbonylamino.

8. A process according to claim 6(a) or 7, characterised in that the acid acceptor is potassium carbonate.

9. A process according to any of claims 6(a), 6(b), 7 or 8, characterised in that the C-8 hydroxy group of the compound of formula (IV) is protected by f rmyl and $R^1$ in formula (VI) is hydrogen.

10. The compound of formula (II) for use in the treatment of a bacterial infection in an animal.

European Patent Office

# EUROPEAN SEARCH REPORT

0079167

Application number

EP 82 30 5687

## DOCUMENTS CONSIDERED TO BE RELEVANT

| Category | Citation of document with indication, where appropriate, of relevant passages | Relevant to claim | CLASSIFICATION OF THE APPLICATION (Int Cl 3) |
|---|---|---|---|
| D,X | EP-A-0 024 832 (BEECHAM) *Pages 47,49; examples 18,20; claims* | 1-9 | C 07 D 487/04<br>A 61 K 31/40 //<br>(C 07 D 487/04<br>C 07 D 209/00<br>C 07 D 205/00 ) |
| A | EP-A-0 001 627 (MERCK) *Claims* | 1,2,6 | |

TECHNICAL FIELDS SEARCHED (Int Cl. 3)

C 07 D 487/00
A 61 K 31/00

The present search report has been drawn up for all claims

| Place of search | Date of completion of the search | Examiner |
|---|---|---|
| THE HAGUE | 02-02-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document